# EUROPEAN PATENT APPLICATION

(11) **EP 4 574 820 A1**
(43) Date of publication of application: **25.06.2025**
(21) Application number: 23855154.3
(22) Date of filing: 16.08.2023
(51) Int. Cl.: C07D 403/04, A61K 9/16, A61K 9/20, A61K 31/506, A61K 9/00

(54) **CRYSTALLINE FORM OF PYRIMIDINE-4-CARBOXAMIDE COMPOUND AND ORAL PHARMACEUTICAL FORMULATION CONTAINING SAME**

(30) Priority: 16.08.2022 KR 20220102292
(71) Applicant: J2H Biotech Inc., Suwon-Si, Gyeonggi-do 16648 (KR)
(72) Inventor: KIM, Jae-Sun, Suwon-Si, Gyeonggi-do 16658 (KR); RYU, Hyung-Chul, Hwaseong-si, Gyeonggi-do 18526 (KR); LIM, Jee-Woong, Seongnam-Si, Gyeonggi-do 13600 (KR); KANG, Hyo Jin, Suwon-si, Gyeonggi-do 16627 (KR); AN, Hong Sun, Cheonan-si, Chungcheongnam-do 31099 (KR); LEE, Kyu Ho, Seongnam-si, Gyeonggi-do 13616 (KR); MIN, Hyun Ki, Seongnam-si, Gyeonggi-do 13616 (KR)
(74) Representative: Maiwald GmbH
(86) International application number: PCT/KR2023/012132
(87) International publication number: WO 2024/039187

(57) **Abstract**

The present invention relates to a novel stable crystalline form of a compound of chemical formula 1 and a method for obtaining such crystalline form. In addition, the present invention relates to a pharmaceutical formulation, especially an oral pharmaceutical formulation, and a preparation method therefor, the pharmaceutical formulation comprising a compound of chemical formula 1 (preferably, a compound of a crystalline form according to the present invention) as an active ingredient and having stability, a high dissolution rate, high bioavailability, etc., secured.

## Description

### [Technical Field]

This application claims the benefit of priority from Korean Patent Application No. 10-2022-0102292, filed on August 16, 2022, and all contents disclosed in the specification and drawings of said application are incorporated herein by reference.

The present invention relates to a novel crystalline form of a pyrimidine-4-carboxamide compound having the specific structure and/or an oral (pharmaceutical) preparation comprising the crystalline solid form as an active ingredient.

### [Background Art]

2-((R)-4-(2-fluoro-4-(methylsulfonyl)phenyl)-2-methylpiperazin-1-yl)-N-((1R,2s,3S,5S, 7S)-5-hydroxyadamantan-2-yl)pyrimidine-4-carboxamide represented by the following chemical formula 1 is a compound included in the chemical formula disclosed in U.S. Patent No. 9,096,571.

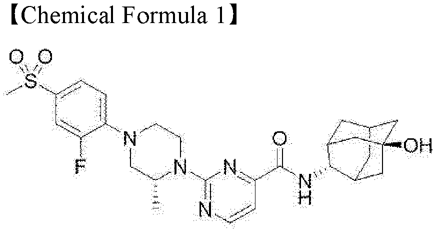

The compound of the above chemical formula 1 is effective in preventing or treating non-alcoholic steatohepatitis, and has the special feature of improving dyslipidemia, protecting liver cells, and exhibiting anti-fibrotic function (see Korean Patent No. 10-2177304).

The compound of the above chemical formula 1 is a substance that falls under the definition of "practically insoluble" with a water solubility of 1 µg/mL or less, and is classified as Class 2 according to the Biopharmaceutics classification system (BCS).

There are various difficulties in developing pharmaceuticals using poorly soluble pharmaceutical raw materials. For example, amorphous or partially crystalline raw materials can be used to improve water solubility, but amorphous or partially crystalline raw materials have the disadvantage of being unstable compared to crystalline raw materials. In addition, amorphous or partially crystalline raw materials may undergo crystal transition during storage or pharmaceutical manufacturing, and it is difficult to control drug dissolution and blood concentration.

Meanwhile, a method of preparing an amorphous solid dispersion using a hydrophilic polymer and/or a water-soluble carrier can be effective in improving the solubility of a drug. However, there is a disadvantage in that the amorphous active ingredient of the solid dispersion changes into a crystalline form depending on the storage conditions, resulting in changes in dissolution. In addition, amorphous solid dispersion formulations have a fundamental problem of lower thermodynamic stability compared to formulations using crystalline raw materials.

### [Disclosure]

### [Technical Problem]

The object that the present disclosure seeks to solve is to provide a novel crystalline form of the compound of the above chemical formula 1 having physicochemical properties useful as an effective ingredient of pharmaceuticals, and a method for producing the crystalline form.

Another object that the present disclosure seeks to solve is to provide a pharmaceutical preparation, preferably an oral preparation, which comprises the compound of the above chemical formula 1 as an effective ingredient, but has secured stability and high bioavailability, and a method for producing the preparation.

### [Technical Solution]

In order to achieve the above object, one embodiment of the present invention provides a crystalline form of the compound (2-((R)-4-(2-fluoro-4-(methylsulfonyl)phenyl)-2-methylpiperazin-1-yl)-N-((1R,2s,3S,5S,7S)-5-h ydroxyadamantan-2-yl)pyrimidine-4-carboxamide) of chemical formula 1, having a powder X-ray diffraction (PXRD) pattern comprising peaks at 2θ diffraction angles of: 5.3±0.2°, 7.5±0.2°, 10.1±0.2°, 10.6±0.2°, 11.5±0.2°, 14.3±0.2°, 14.7±0.2°, 15.0±0.2°, 15.5±0.2°, 16.0±0.2°, 16.3±0.2°, 16.5±0.2°, 19.1±0.2°, 19.3±0.2°, 19.7±0.2°, 20.3±0.2°, 20.6±0.2°, 21.4±0.2°, 21.8±0.2°, 22.6±0.2°, and 27.5±0.2°.

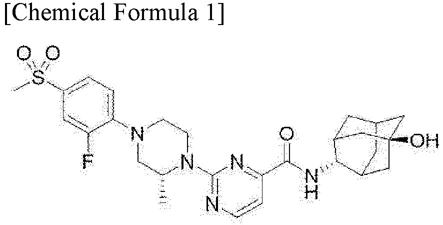

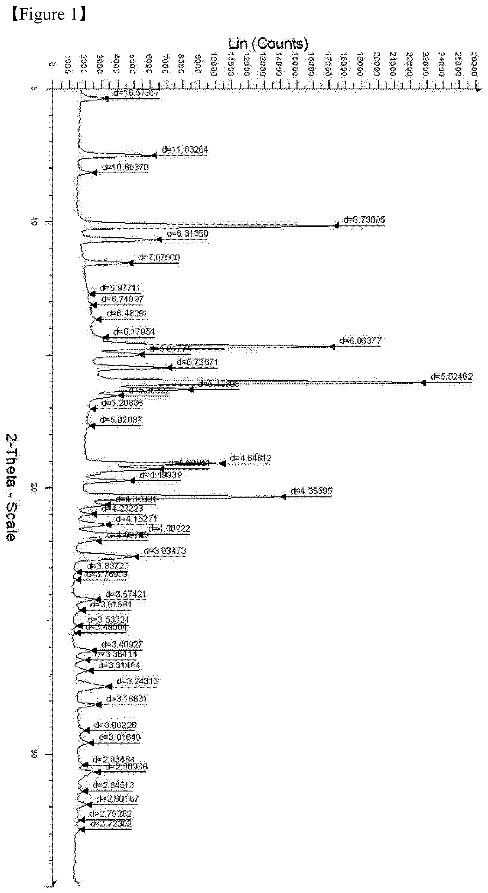

In another embodiment of the present invention, the present invention provides a crystalline form of the compound of chemical formula 1 having an X-ray diffraction pattern as shown in figure 1.

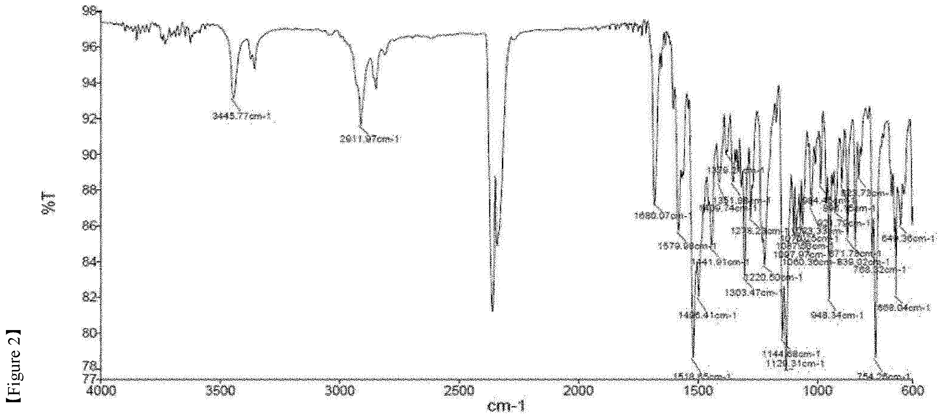

In another embodiment of the present invention, the present invention provides a crystalline form which exhibits absorption patterns of 1100 cm⁻¹ (aromatic C-F), 1518 cm⁻¹ (NH), 1579 cm⁻¹ (aromatic C=C), 1680 cm⁻¹ (C=O), 2911 cm⁻¹ (aliphatic C-H), and 3445 cm⁻¹ (OH) in infrared absorbance analysis. In yet another embodiment of the present invention, the present invention provides a crystalline form of the compound of chemical formula 1 having an absorption pattern as shown in figure 2 in infrared absorbance analysis.

Even compounds with the same chemical formula exhibit different physicochemical properties depending on the crystal form. Differences depending on the crystal form include, but are not limited to, stability (e.g., heat or light stability), compressibility and density (important in terms of formulation and production), and solubility (which may affect bioavailability). In particular, differences in stability may cause changes in chemical reactivity (e.g., discoloration, moisture absorption) or mechanical properties (e.g., changes due to conversion to a thermodynamically more stable polymorph). Specifically, humidity stability may vary depending on the crystal form, the possibility of forming a soluble compound may vary, and the ease of filtration or washing may also vary.

The inventors of the present invention have been able to secure a stable crystal form of the compound of chemical formula 1 according to the present invention through much effort, and the crystal form according to the present invention exhibits desirable physicochemical and mechanical properties in various aspects mentioned above. In particular, the crystal form according to the present invention is very stable, so there is no concern that the physicochemical properties of the pharmaceutical ingredient will change during the storage of raw materials, production of pharmaceuticals, storage and distribution of pharmaceuticals.

Another embodiment of the present invention also provides a method for preparing a crystalline form of the compound of formula 1 according to the present invention, comprising the steps of (S1) dissolving the compound of chemical formula 1 in ethyl acetate; (S2) adding t-butyl methyl ether to the ethyl acetate solution of step S1 to crystallize; and (S3) filtering the suspension of step S2 to obtain a crystalline compound of formula 1.

In addition, the present inventors attempted to prepare an oral preparation using the compound of chemical formula 1, particularly the crystalline form according to the present invention, as a pharmaceutical raw material. The compound of chemical formula 1 is a substance that falls within the definition of "practically insoluble" with a water solubility of 1 µg/mL or less, and is a poorly soluble substance classified as Class 2 (high permeability, low solubility) according to the Biopharmaceutics classification system (BCS).

In order to increase the bioavailability of the poorly soluble substance, the present inventors have made various attempts. First, since the compound of chemical formula 1 does not have the characteristic of improving solubility according to pH, there was no room to control the dissolution rate of the drug by making a salt. In addition, it was not easy to achieve long-term stability when using an amorphous raw material or a solid dispersion technology in which the active ingredient exists in an amorphous state. In addition, it was difficult to achieve the desired level of bioavailability even by simply mixing hydrophilic components (e.g., hydrophilic polymers, surfactants).

In this situation, the present inventors confirmed that when a pharmaceutical preparation is manufactured by drying water in which the compound of chemical formula 1 is suspended and the hydrophilic polymer is dissolved, not only can (long-term) stability be achieved, but also bioavailability can be dramatically increased.

Therefore, another embodiment of the present invention also provides a pharmaceutical preparation, preferably an oral pharmaceutical preparation, comprising particles prepared by drying water in which the compound of the above chemical formula 1 (preferably, the compound having the crystal form according to the present invention) is suspended and a hydrophilic polymer is dissolved.

In a preferred embodiment of the present invention, the hydrophilic polymer may be at least one selected from the group consisting of hypromellose, polyvinylpyrrolidone, polyvinyl alcohol, and hydroxypropylcellulose, and among these, hypromellose is more preferred for the purpose of the present invention.

In terms of achieving the purpose of the present invention, the hydrophilic polymer is preferably included in an amount of 30 to 80 wt% relative to the content of the compound of the chemical formula 1, and more preferably in an amount of 40 to 70 wt% relative to the content of the compound of the chemical formula 1.

Another embodiment of the present invention also provides a pharmaceutical preparation, preferably an oral pharmaceutical preparation, comprising particles prepared by drying water in which a micronized compound of chemical formula 1 (preferably a compound having the crystalline form according to the present invention) is suspended and a hydrophilic polymer is dissolved. The type and content of the hydrophilic polymer are the same as mentioned above.

Preferably, the micronized compound of chemical formula 1 has a d(50) of 5 µm or less, and more preferably, a d(50) of 2 µm or less.

Such micronized compound of chemical formula 1 can be performed using jet milling, a high-pressure particle size grinder (Microfluidics), etc., but the present invention is not limited to such specific micronization means.

The bioavailability of the compound of chemical formula 1 can be dramatically increased by a specific manufacturing method using the hydrophilic polymer, and in particular, the bioavailability can be further increased by using the micronized compound of chemical formula 1 (preferably, the compound having the crystalline form according to the present invention). In the pharmaceutical preparation of the present invention, the hydrophilic polymer is thought to have various roles such as stabilizing the micronized compound of chemical formula 1 dispersed in the suspension phase so that it does not aggregate and become larger again, and enabling it to be well wetted in an aqueous solution by being bound to the surface of the compound of chemical formula 1, thereby exhibiting a synergistic effect. However, the present invention is not limited to such theoretical predictions. In particular, the effect of the present invention is closely related to the manufacturing method in that it is difficult to achieve the desired bioavailability by simply mixing the relevant components.

In a preferred embodiment of the present invention, the process of micronizing the compound of chemical formula 1 may be carried out in water in which the compound of chemical formula 1 (preferably, the compound having the crystalline form according to the present invention) is suspended and the hydrophilic polymer is dissolved. That is, in a preferred embodiment of the present invention, the micronization process of the compound of formula 1 is performed after mixing with the hydrophilic polymer.

Another embodiment of the present invention also provides a pharmaceutical preparation, preferably an oral pharmaceutical preparation, comprising particles prepared by drying water in which a compound of chemical formula 1 (preferably a compound having the crystalline form according to the present invention and micronized) is suspended and a hydrophilic polymer and a surfactant are dissolved. The type and content of the hydrophilic polymer and the micronization-related technology are the same as mentioned above.

The surfactant is preferably at least one selected from the group consisting of lauryl sulfate salt, poloxamer, glycerol monostearate, polyoxyethylene, and docusate salt, and sodium lauryl sulfate is particularly preferred for the purpose of the present invention.

It is preferred that such surfactant is included in the pharmaceutical preparation in an amount of 0.2 to 2 wt% relative to the total weight of the pharmaceutical preparation.

The particles prepared by drying water in which a compound of chemical formula 1 (preferably a compound having the crystalline form according to the present invention and micronized) is suspended and the hydrophilic polymer and optionally the surfactant are dissolved may be prepared as capsules by mixing with other carriers (e.g., diluents), lubricants, etc. and filling into empty capsules, or may be prepared as tablets by mixing with other carriers, lubricants, etc. and then compressed. In a preferred embodiment of the present invention, the pharmaceutical formulation is a tablet.

The particles can be prepared by spraying onto other carriers and then drying water in which a compound of chemical formula 1 (preferably a compound having the crystalline form according to the present invention and micronized) is suspended and the hydrophilic polymer and optionally the surfactant are dissolved

The carrier that can be used in the present invention, for example, as a diluent, microcrystalline cellulose, lactose, lactose hydrate, anhydrous lactose, mannitol, starch, dihydrogenated calcium phosphate, anhydrous dibasic calcium phosphate, etc. can be used. In a preferred embodiment of the present invention, the carrier is microcrystalline cellulose, lactose hydrate, mannitol, or a mixture thereof. In a preferred embodiment of the present invention, the carrier is microcrystalline cellulose and lactose hydrate. In a preferred embodiment of the present invention, the carrier is microcrystalline cellulose, lactose hydrate, and mannitol.

The pharmaceutical preparation of the present invention may also optionally further comprise a disintegrant. The disintegrant may be, for example, sodium starch glycolate, crospovidone, croscarmellose sodium, maltodextrin, or a mixture thereof. In a preferred embodiment of the present invention, the disintegrant is crospovidone.

The pharmaceutical preparation of the present invention may optionally further comprise a lubricant. The lubricant is used to ensure smooth flow during capsule filling, packaging, etc., or to prevent sticking, capping, etc. during tablet compression. Suitable lubricants include colloidal silica, talc, magnesium stearate, or mixtures thereof. In a preferred embodiment of the present invention, the lubricant is magnesium stearate.

### [Advantageous Effects]

One embodiment of the present invention provides a stable novel crystalline form of the compound of chemical formula 1 and a method for obtaining such a crystalline form. Another embodiment of the present invention also provides a pharmaceutical preparation, preferably an oral pharmaceutical preparation, comprising the compound of chemical formula 1 (preferably the crystalline compound according to the present invention) as an active ingredient and ensuring stability, high dissolution rate, high bioavailability, etc., and a method for preparing the same.

### [Brief Description of Figures]

Figure 1 is a result of powder X-ray diffraction pattern (PXRD) evaluation for the crystalline compound prepared in Example 1.
Figure 2 is a result of infrared spectroscopy (IR) absorbance measurement for the crystalline compound prepared in Example 1.
Figure 3 is a graph comparing blood concentrations obtained after oral administration of tablets prepared in Preparation Example 10, which is one embodiment of the present invention, to beagle dogs with comparative examples.

### [Mode for Invention]

Hereinafter, in order to help understand the present invention, examples and the like will be described in detail. However, the examples according to the present invention may be modified in various different forms, and the scope of the present invention should not be construed as being limited to the following examples. The examples of the present invention are provided to more completely explain the present invention to a person having average knowledge in the field to which the present invention pertains.

### Example 1: Preparation of crystalline solid (crystal form) of compound of chemical formula 1

4.14 kg of compound of chemical formula 1 in solid form was dissolved in 17 kg of ethyl acetate, stirred at room temperature, and 15.5 kg of t-butyl methyl ether was gradually added. The resultant was stirred at 25°C for 3 hours to obtain a homogeneous suspension, which was filtered, and washed with 2.5 kg of t-butyl methyl ether. The resultant was vacuum-dried at 45°C for 15 hours, and 3.52 kg of a white crystalline solid was obtained. (Yield 85%)

¹H NMR (400MHz, DMSO-d6) δ 8.64 (d, J = 4.8 Hz, 1H), 8.20 (d, J = 7.8 Hz, 1H), 7.68 (dd, J = 2.2, 12.4 Hz, 1H), 7.65 (dd, J = 2.2, 8.5 Hz, 1H), 7.25 (t, J = 8.5 Hz, 1H), 7.16 (d, J = 4.8 Hz, 1H), 4.93-4.88 (m, 1H), 4.56-4.52 (m, 1H), 4.49 (s, 1H), 3.96-3.93 (m, 1H), 3.67-3.63 (m, 1H), 3.59-3.56 (m, 1H), 3.41-3.37 (m, 1H), 3.20 (s, 3H), 3.14-3.11 (m,1H), 3.01-2.98 (m, 1H), 2.10-2.05 (m, 3H), 1.78-1.72 (m, 4H), 1.68-1.63 (m, 4H), 1.49-1.44 (m, 2H), 1.29 (d, J = 6.7 Hz, 3H); MS (FAB) m/z: 544 [M + H]+.

In order to obtain a suitable crystalline form, in addition to the combination condition of ethyl acetate/t-butyl methyl ether of Example 1, other various combinations (for example, using ethyl ether, isobutyl acetate, isopropyl acetate, methyl isobutyl ketone, n-butyl acetate, n-heptane, n-pentane, n-hexane, cyclohexane, methylcyclohexane, tetrahydrofuran, acetone, etc. instead of t-butyl methyl ether) were tried, but no crystalline form with satisfactory stability was obtained.

Meanwhile, t-butyl methyl ether was fixed, and instead of ethyl acetate, solvents such as acetonitrile, dichloromethane, 1,2-dichloroethane, 1,2-dimethoxyethane, N,N-dimethylacetamide, 1,4-dioxane, methanol, ethanol, ethylene glycol, 1-butanol, 2-butanol, 1-propanol, 2-propanol, and propyl acetate were tried. However, a sufficiently stable crystalline form as a pharmaceutical raw material was not obtained.

In addition, the above ethyl acetate/t-butyl methyl ether combination was changed, and various combinations of solubilizing solvents and antisolvents were tried to prepare the crystalline form, but the target crystalline form could not be obtained.

In preparing the crystalline form of a pharmaceutical raw material, the selection of an appropriate single solvent or mixed solvent is very important. The resulting crystalline solid must have excellent stability and desirable physical properties, and after drying the crystalline solid, it must meet the residual solvent allowance standard of the ICH guideline (GUIDELINE FOR RESIDUAL SOLVENTS Q3C(R8)). For this reason, solvates, excluding hydrates, are often not usable as pharmaceutical raw materials even if they actually have excellent crystallinity.

The present inventors attempted to precipitate a solid using the solvent evaporation method after dissolving the compound of chemical formula 1 using a single solvent as solubilizing solvent(s), but the crystalline form obtained in this way had a problem in that it was a solvate or the residual solvent was not sufficiently removed even after a long period of reduced pressure drying, so that it greatly exceeded the ICH standard. In methods of inducing precipitation of solids using various solubilizing solvents and antisolvents, the obtained results were mainly amorphous, solvated, or other crystalline forms that could not be used as raw materials for medicines because the residual solvent was not easily removed. Even then, the yield was poor or discoloration occurred during the manufacturing process.

That is, the present inventors confirmed that the combination of ethyl acetate/t-butyl methyl ether as a mixed solvent for preparing the crystalline form of the above chemical formula 1 having suitable properties as a raw material for medicine is suitable for the purpose of the present invention. The crystalline solid thus obtained not only has excellent stability and physical properties, but is also an anhydrous crystalline form, and the residual solvent analysis results did not exceed the residual limits allowed by the ICH guidelines for each of the above solvents, so it was sufficient for use as a raw material for medicine.

### Experimental Example 1: Powder X-ray diffraction (PXRD) analysis of the crystalline form of Example 1

A powder X-ray diffraction pattern is a unique characteristic of a crystalline form and is widely used to distinguish polymorphism and hydrates.

The crystallinity analysis of the crystalline compound produced in Example 1 was performed on an X-ray powder diffractometer using Cu-Kα radiation. The measuring equipment was equipped with a reactive power, and the current was set to 45 kV and 40 mA. The divergence and scattering slits were set to 1°, and the receiving slit was set to 0.2 mm. A θ-2θ continuous scan at 3°/min (0.4 s/0.02° step) was used from 5° to 35° 2θ.

The experimental results are shown in figure 1. As disclosed in figure 1, the crystalline compound prepared according to Example 1 has a powder X-ray diffraction (PXRD) pattern comprising peaks at 2θ diffraction angles of: 5.3±0.2°, 7.5±0.2°, 10.1±0.2°, 10.6±0.2°, 11.5±0.2°, 14.3±0.2°, 14.7±0.2°, 15.0±0.2°, 15.5±0.2°, 16.0±0.2°, 16.3±0.2°, 16.5±0.2°, 19.1±0.2°, 19.3±0.2°, 19.7±0.2°, 20.3±0.2°, 20.6±0.2°, 21.4±0.2°, 21.8±0.2°, 22.6±0.2°, and 27.5±0.2°.

### Experimental Example 2: Infrared spectroscopy (IR) analysis of the crystalline form of Example 1

The vibrational energy between atoms that constitute the molecules of a substance in a crystalline solid varies depending on the crystal structure, which means that different crystalline forms exhibit different infrared absorbances. Therefore, the infrared absorbance of a specific crystalline material can be considered to reflect the unique characteristics of the crystalline form.

The infrared absorbance of the crystalline form prepared by Example 1 was analyzed using Perkin Elmer FT-IR (fourier transform infrared spectroscopy), and the results are shown in figure 2. As disclosed in figure 2, the absorption patterns were shown at 1100 cm⁻¹ (aromatic C-F), 1518 cm⁻¹ (NH), 1579 cm⁻¹ (aromatic C=C), 1680 cm⁻¹ (C=O), 2911 cm⁻¹ (aliphatic C-H), and 3445 cm⁻¹ (OH).

### Experimental Example 3: Stability evaluation for the crystalline form of Example 1

The compound of chemical formula 1 is a raw material used for pharmaceutical purposes, and appropriate specifications and stability must be secured in order to set the storage method and period of use of the pharmaceutical. In other words, the stability of the raw pharmaceutical material is one of the most important factors for maintaining the quality of the final product.

The stability of the crystalline solid of the compound of chemical formula 1 prepared by Example 1 was evaluated. According to the ICH guideline, the stability test was conducted under long-term storage (25±2°C, 60±5% RH) and accelerated (40±2°C, 75±5% RH) conditions. The analysis was performed using HPLC to evaluate the change in content and the occurrence of impurities, and the results are shown in Table 1 below.

### Stability Evaluation Results of the Crystalline Compound of Chemical Formula 1 (HPLC, %)

**[Table 1]**

| | Test items | Test criteria | Initial | 1 months | 3 months | 6 months | 12 months |
|---|---|---|---|---|---|---|---|
| Long-term storage condition (25±2°C, 60±5% RH) | Content | > 99.0% | 99.9 | 99.5 | 99.3 | 100.5 | 99.7 |
| | Isomers | < 0.5% | Not detected | Not detected | Not detected | Not detected | Not detected |
| | Total impurities | < 1.5% | 0.34 | 0.43 | 0.41 | 0.43 | 0.45 |
| Accelerated condition (40±2°C, 75±5% RH) | Content | > 99.0% | 99.9 | 99.6 | 100.8 | 100.0 | - |
| | Isomers | < 0.5% | Not detected | Not detected | Not detected | Not detected | - |
| | Total impurities | < 1.5% | 0.34 | 0.43 | 0.44 | 0.42 | - |

As can be seen from the results in Table 1 above, the crystalline solid compound of chemical formula 1 of the present invention has excellent stability with almost no change in the content and no increase of impurities for up to 12 months and 6 months, respectively, when stored under long-term and accelerated storage conditions.

In addition, the crystalline form of the sample stored for 3, 6, and 12 months (in the case of long-term storage test) of the stability test was measured in the same manner as in Experimental Example 1, and it was confirmed that the crystalline form did not change. For a BCS Class 2 substance such as the compound of chemical formula 1, it is very important that the crystalline form remains stable under storage conditions. The more insoluble a substance is, the greater the impact of changes in crystallinity on the quality of the final product is. The change in the crystalline form causes changes in hygroscopicity, stability, dissolution, etc. Sometimes, the quality of the final product can be significantly reduced even by partial crystalline transition in which only a part of the pharmaceutical raw material, not the entirety, changes. The crystalline form of the present invention is suitable for use as a raw material for medicine because it does not undergo polymorphic transition over time.

### Preparation examples 1 to 3 of final products: Manufacturing formulations having different particle sizes of the compound of chemical formula 1

Preparation examples 1 to 3, which are compositions comprising the crystalline form of Example 1, were manufactured according to Table 2 below. In the case of Preparation examples 2 and 3, the compositions were all completely the same, and only the manufacturing method was different to prepare formulations having different particle sizes.

**[Table 2]**

| Classification (Unit: mg) | Ingredient name | Preparation Example 1 | Preparation Example 2 | Preparation Example 3 |
|---|---|---|---|---|
| Binding solution | Chemical formula 1 Compound | 100 | 100 | 100 |
| | Hypromellose | | 15 | 15 |
| | Sodium lauryl sulfate | | 1.5 | 1.5 |
| | Purified water | | 500 | 500 |
| Carrier | Microcrystalline cellulose | | 250 | 250 |
| | Lactose hydrate | | 250 | 250 |

First, in the composition of Table 2, Preparation Example 1 did not apply any treatment to the main ingredient, chemical formula 1 compound.

In Preparation Example 2, those ingredients used in the binding solution were mixed and then homogenized using a homogenizer to prepare a binding solution comprising particles of chemical formula 1 compound.

In Preparation Example 3, those ingredients used in the binding solution were mixed and then pulverized under high-energy milling conditions using a high-pressure particle size grinder (Microfluidics) to prepare a binding solution comprising micronized particles of chemical formula 1 compound.

The particle sizes of chemical formula 1 compound in Preparation Examples 2 and 3 were measured in the state of the binding solution, that is, in the state where SLS and HPMC were mixed. Since the binding solution was in a suspended state, SLS and HPMC were dissolved in purified water, and only the main ingredient was suspended in a solid state, it was possible to measure the particle size of the main ingredient. The particle size of the chemical formula 1 compound was measured using a laser scattering particle size analyzer (Mastersizer 2000, manufacturer: Malvern Instruments). The particles of the chemical formula 1 compound manufactured according to Preparation Examples 1 to 3 and the solvent (purified water) were added to a beaker (1,000 ml), stirred at 1,500 rpm, and the particles in the homogeneously suspended dispersion phase were measured. The results are shown in Table 3.

### <Conditions for measuring particle size>

Device : Malvern Mastersizer 2000 / Hydro 2000MU
Sample Amount : 0.5g
Sample refractive index : 1.520
Interpretation model : General purpose
Sensitivity : Normal
Sample measurement time : 10 seconds
Interpretation range : 0.020 - 2000.0 µm

**[Table 3]**

| Sample | Particle size (µm) | | |
|---|---|---|---|
| | d(10) | d(50) | d(90) |
| Preparation Example 1 | 1.12 | 4.90 | 16.01 |
| Preparation Example 2 | 1.83 | 4.59 | 9.28 |
| Preparation Example 3 | 0.10 | 0.45 | 2.52 |

In the case of Preparation Examples 2 to 3, granules were manufactured using a fluid bed granulator. The binding solution was sprayed onto microcrystalline cellulose and lactose hydrate used as carriers, and the granules were manufactured under the conditions of an injection temperature of 70-85°C, an internal temperature of 35-45°C, and an injection speed of 6-30 g/min. The main ingredient or granules prepared in Preparation Examples 1 to 3 were filled into capsules.

### Experimental Example 4: Effect on dissolution rate according to particle size

The formulations of Preparation Examples 1 to 3 were tested using the 2^{nd} method of the Korean Pharmacopoeia Dissolution Test (Paddle Method). The dissolution was performed using purified water, a paddle speed of 50 rpm, and at 37°C, and quantified using HPLC (manufacturer: Agilent) under the following conditions. The results are shown in Table 4.

### <HPLC Measurement Conditions>

Detector : UV spectrophotometer (measuring wavelength : 254 nm)
Column : Inertsil ODS-4 C18 (4.6 X 150 mm, 5 µm)
Flow rate : 1.0 mL/min
Column temperature : 35°C
Mobile phase : pH3.0 phosphoric acid solution: acetonitrile = 4:6 (v/v%)
Injection amount : 20 µL

**[Table 4]**

| Sample/Time (minutes) | Dissolution rate (%) | | | | | |
|---|---|---|---|---|---|---|
| | 0 minute | 5 minutes | 10 minutes | 15 minutes | 30 minutes | 45 minutes |
| Preparation Example 1 | 0 | 0.74 | 1.72 | 3.51 | 5.02 | 5.52 |
| Preparation Example 2 | 0 | 1.51 | 5.34 | 6.63 | 8.13 | 9.04 |
| Preparation Example 3 | 0 | 3.67 | 10.25 | 9.84 | 9.79 | 9.95 |

As can be seen in Table 4 above, the dissolution speed and dissolution rate were affected by particle size, and it was evaluated that the dissolution rate increased as the particle size decreased. This is presumed to be because the surface area increases as the particle size decreases, but the present invention is not limited to this theoretical mechanism. In terms of the dissolution rate at 45 minutes, the dissolution rates of Preparation Examples 2 to 3, where the particle size was reduced, increased by about twice compared to Preparation Example 1. In addition, it was determined that additional use of a hydrophilic polymer was necessary to improve the dissolution rate, and thus an evaluation was conducted.

### Preparation Examples 4 to 6: Dissolution changes according to the content of hydrophilic polymers

In order to compare and evaluate the dissolution effects according to the content of hydrophilic polymers, tablets of Preparation Examples 4 to 6 were prepared with the compositions shown in Table 5 below. Preparation Example 4 used the hydrophilic polymer only in the binding solution 1, and Preparation Examples 5 to 6 additionally used the binding solution 2 in which the hydrophilic polymer was dissolved.

**[Table 5]**

| Classification (Unit: mg) | Ingredient name | Preparation Example 4 | Preparation Example 5 | Preparation Example 6 |
|---|---|---|---|---|
| Binding solution 1 | Chemical formula 1 Compound | 100 | 100 | 100 |
| | Hypromellose | 15 | 15 | 15 |
| | Sodium lauryl sulfate | 1.5 | 1.5 | 1.5 |
| | Purified water | 500 | 300 | 300 |
| Binding solution 2 | Hypromellose | 0 | 35 | 45 |
| | Purified water | 0 | 200 | 200 |
| Carrier | Microcrystalline cellulose | 250 | 250 | 250 |
| | Lactose hydrate | 250 | 250 | 250 |
| Post-mix | D-mannitol | 50 | 50 | 50 |
| | Crospovidone | 36.5 | 36.5 | 36.5 |
| | Magnesium stearate | 5 | 5 | 5 |

Preparation Example 4 was manufactured as follows: granules were made using the method of Preparation Example 3, and then the granules were mixed well with mannitol, crospovidone, and magnesium stearate, and then compressed into tablets.

Preparation Examples 5 to 6 were manufactured as follows: the binding solution 1 was prepared using the method of Preparation Example 3, and the binding solution 2 was prepared by mixing hypromellose and purified water. Then, the binding solution 1 and the binding solution 2 were mixed to prepare the final binding solution. Then, granules were manufactured according to the method of Preparation Example 3 using microcrystalline cellulose and lactose hydrate as carriers. The manufactured granules were mixed well with mannitol, crospovidone, and magnesium stearate, and then compressed into tablets.

The particle size of the chemical formula 1 compound manufactured according to Preparation Examples 4 to 6 was measured using a laser scattering particle size analyzer (Mastersizer 2000, manufacturer: Malvern Instruments). The results are shown in Table 6 below.

**[Table 6]**

| Sample | Particle size (µm) | | |
|---|---|---|---|
| | d(10) | d(50) | d(90) |
| Preparation Example 4 | 0.10 | 0.45 | 2.52 |
| Preparation Examples 5 to 6 | 0.10 | 0.63 | 3.76 |

### Experimental Example 5: Changes in dissolution according to the content of hydrophilic polymer

Similar to Experimental Example 4, tablets of Preparation Examples 4 to 6 were tested by the 2^{nd} dissolution test method (paddle method) of the Korean Pharmacopoeia. The dissolution solution was purified water, paddle speed 50 rpm, and dissolution test was performed at 37°C. The quantification was performed by HPLC (manufacturer: Agilent) under the same conditions as Experimental Example 4. The results are shown in Table 7.

**[Table 7]**

| Sample/Time (minutes) | Dissolution rate (%) | | | | | | |
|---|---|---|---|---|---|---|---|
| | 0 min | 5 mins | 10 mins | 15 mins | 30 mins | 45 mins | 60 mins |
| Preparation Example 4 | 0 | 1.9 | 5.8 | 7.2 | 8.6 | 9.7 | **10.3** |
| Preparation Example 5 | 0 | 10.9 | 33.5 | 51.0 | 58.4 | 60.0 | **52.6** |
| Preparation Example 6 | 0 | 8.5 | 24.1 | 30.9 | 45.8 | 51.3 | **48.9** |

As shown in Table 7, the dissolution rate of Preparation Example 4, in which the amount of hydrophilic polymer (hypromellose) was 15 mg (15 parts by weight relative to 100 weight of the chemical formula 1 compound) per tablet, did not increase much. However, in Preparation Examples 5 to 6, in which the amount of hydrophilic polymer was 50 mg to 60 mg (50-60 parts by weight relative to 100 weight of the chemical formula 1 compound) per tablet, the dissolution rate increased by about 5 to 6 times compared to Preparation Example 4.

### Preparation Examples 7 to 10: Dissolution changes according to the amount of surfactant

The effects on dissolution according to the amount of surfactant were compared and evaluated. The tablets of Preparation Examples 7 to 10 were prepared with the compositions shown in Table 8 below.

**[Table 8]**

| Classification (Unit: mg) | Ingredient name | Preparation Example 7 | Preparation Example 8 | Preparation Example 9 | Preparation Example 10 |
|---|---|---|---|---|---|
| Binding solution 1 | Chemical formula 1 Compound | 100 | 100 | 100 | 100 |
| | Hypromellose | 15 | 20 | 20 | 15 |
| | Sodium lauryl sulfate | 1.5 | 8 | 1 | 1 |
| | Purified water | 300 | 450 | 250 | 250 |
| Binding solution 2 | Hypromellose | 35 | 30 | 30 | 35 |
| | Sodium lauryl sulfate | | | 7 | 7 |
| | Purified water | 200 | 200 | 250 | 250 |
| Carrier | Microcrystalline cellulose | 250 | 100 | 100 | 100 |
| | Lactose hydrate | 250 | 100 | 100 | 100 |
| Post-mix | D-mannitol | 67 | 67 | 67 | 67 |
| | Crospovidone | 50 | 50 | 50 | 50 |
| | Magnesium stearate | 5 | 5 | 5 | 5 |

Preparation Examples 7 to 10 were manufactured according to the method of Preparation Example 6. The amount of surfactant was 1.5 to 8 mg, and the difference according to the amount of surfactant and the manufacturing method was evaluated by using each of the binding solution 1 and binding solution 2.

The particle size of the chemical formula 1 compound manufactured according to Preparation Examples 7 to 10 was measured using a laser scattering particle size analyzer (Mastersizer 2000, manufacturer: Malvern Instruments). The results are shown in Table 9 below.

**[Table 9]**

| Sample | Particle size (µm) | | |
|---|---|---|---|
| | d(10) | d(50) | d(90) |
| Preparation Example 7 | 0.10 | 0.63 | 3.31 |
| Preparation Example 8 | 0.47 | 1.39 | 2.09 |
| Preparation Example 9 | 0.61 | 1.51 | 3.67 |
| Preparation Example 10 | 0.37 | 1.62 | 2.73 |

### Experimental Example 6: Dissolution changes according to surfactant content

The tablets of Preparation Examples 7 to 10 were tested by the 2^{nd} Dissolution Test Method (Paddle Method) of the Korean Pharmacopoeia. The dissolution solution was purified water (0.3 wt% sodium lauryl sulfate (SLS) was added), the paddle speed was 50 rpm, and the dissolution test was performed at 37°C. The quantification was performed by HPLC (Manufacturer: Agilent) under the same conditions as the previous method. The results are shown in Table 10 below.

**[Table 10]**

| Sample/Time (minutes) | Dissolution rate (%) | | | | | | |
|---|---|---|---|---|---|---|---|
| | 0 min | 5 mins | 10 mins | 15 mins | 30 mins | 45 mins | 60 mins |
| Preparation Example 7 | 0 | 11.2 | 27.8 | 42.2 | 68.8 | 86.9 | 98.3 |
| Preparation Example 8 | 0 | 28.3 | 45.9 | 63.2 | 93.1 | 99.7 | 100.1 |
| Preparation Example 9 | 0 | 15.2 | 35.5 | 50.2 | 79.4 | 100.1 | 105.2 |
| Preparation Example 10 | 0 | 24.2 | 47.6 | 67.5 | 97.1 | 97.7 | 97.7 |

As shown in Table 10 above, the final dissolution rate reached in 60 minutes was similar regardless of the amount of surfactant changed from 1.5 mg to 8 mg per tablet.

### Experimental Example 7: Stability test of formulation

For Preparation Example 10, the content and impurities were evaluated after storage under accelerated (40±2°C, 75%RH±5%) and long-term conditions (25±2°C, 60%RH±5%). The results are shown in Tables 12 to 13. The unit is %.

### <HPLC Measurement Conditions_Content>

Detector: UV Spectrophotometer (Measurement Wavelength: 254 nm)
Column: Inertsil ODS-4 C18 (4.6 X 150 mm, 5 µm)
Flow Rate: 1.0 mL/min
Column Temperature: 35°C
Mobile Phase: pH3.0 Phosphoric Acid Solution: Acetonitrile = 4:6 (v/v%)
Injection Volume: 20 µL
Measurement Range: 7 minutes

### <HPLC Measurement Conditions_Purity>

Detector: UV Spectrophotometer (Measurement Wavelength: 254 nm)
Column: YMC Hydrosphere C18 5 µm, 4.6 x 250 mm
Flow Rate: 1.0 mL/min
Column Temperature: 35°C
Mobile Phase A: 0.68 g of potassium dihydrogen phosphate was dissolved in water to make 1000 mL, and the pH was adjusted to 3.0 with phosphoric acid.
Mobile Phase B: Acetonitrile and methanol were mixed in a ratio of 95:5.
Measurement Range: 60 minutes
Gradient Elution Conditions:

**[Table 11]**

| Time (minutes) | Mobile Phase A (%) | Mobile Phase B (%) |
|---|---|---|
| 0 | 80 | 20 |
| 20 | 30 | 70 |
| 40 | 20 | 80 |
| 45 | 20 | 80 |
| 50 | 80 | 20 |
| 60 | 80 | 20 |

**[Table 12]**

| Content (%) | Initial | Long term 1 month | Long term 3 months | Long term 6 months | Long term 9 months | Long term 12 months |
|---|---|---|---|---|---|---|
| Preparation Example 10 | 98.04 | 100.34 | 98.25 | 98.07 | 98.27 | 98.02 |
| Impurities (%) | Initial | Long term 1 month | Long term 3 months | Long term 6 months | Long term 9 months | Long term 12 months |
| Preparation Example 10 | 0.24 | 0.23 | 0.23 | 0.24 | 0.22 | 0.23 |

**[Table 13]**

| Content (%) | Accelerated condition 1 month | Accelerated condition 3 months | Accelerated condition 6 months |
|---|---|---|---|
| Preparation Example 10 | 100.65 | 98.21 | 98.17 |
| Impurities (%) | Accelerated condition 1 month | Accelerated condition 3 months | Accelerated condition 6 months |
| Preparation Example 10 | 0.23 | 0.23 | 0.24 |

As shown in Tables 12 and 13 above, the formulation of the present invention was confirmed to be stable without significant changes as a result of the content evaluation and purity test during the stability test period.

### Experimental Example 8: Comparative pharmacokinetic test of the formulation

By applying the conditions of Preparation Example 10, tablets containing 25, 50, and 100 mg of the crystalline solid of chemical formula 1 as a pharmaceutical raw material were manufactured. An experiment was conducted to analyze the pharmacokinetic characteristics of the manufactured tablets after oral administration to a beagle dog.

The compound of chemical formula 1 was administered orally to a beagle dog as a single dose of 25, 50, and 100 mg tablets/head, orally as a single dose of 25 mg liquid/head, and intravenously as a single dose of 2 mg/kg. Blood samples were collected before (0) and 15, 30 min, 1, 1.5, 2, 4, 6, 8, 10, 16, and 24 hr after administration of the test substance to measure the blood concentration of the compound of chemical formula 1.

The preparation for single oral administration in liquid/head was prepared by homogeneously suspending 275 mg of the compound of chemical formula 1 in 220 ml of pre-prepared 2.5% HP-βCD (sterile water solution) to obtain 1.25 mg/ml. In addition, the preparation for single intravenous administration was prepared by dissolving 300 mg of the compound of chemical formula 1 in 150 ml of pre-prepared 10% HP-βCD (sterile water solution) to obtain 2 mg/ml.

As a result of observing general symptoms after administration, no abnormal symptoms due to the administration of the test substance were observed during the entire experimental period.

It was confirmed from the analytical method validation results in dog blood plasma that there was no influence of interference substances, and it showed good linearity in the concentration range of 5 to 5000 ng/mL (plasma) and uniform results of intra- and inter-test accuracy and precision. It showed sufficient selectivity, linearity, accuracy, and precision that can be applied to sample analysis.

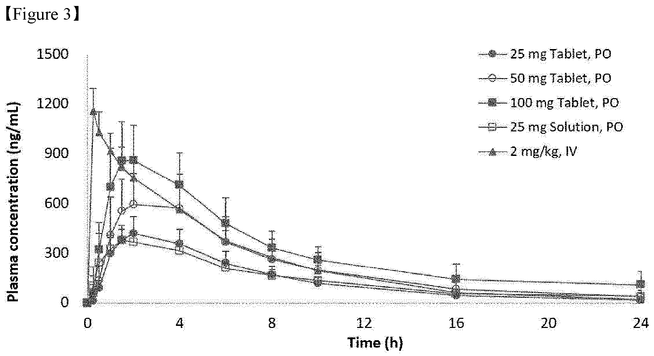

The drug concentration-time curve analyzed in plasma obtained by administering the tablets and liquid chemical formula 1 compound and the pharmacokinetic parameters calculated using the BA Calc 2007 program showed the profiles as shown in figure 3 and Table 14.

**[Table 14]**

| Dosage (mg/head) | Pharmacokinetic parameters | | | | | | |
|---|---|---|---|---|---|---|---|
| | AUCₗₐₛₜ (ng·hr/mL) | Cₘₐₓ (ng/mL) | Tₘₐₓ (hr) | CL(_{inf})/F (L/hr/kg) | Vz(ₜₑᵣₘᵢₙₐₗ)/ F (L) | t_{1/2} (hr) | BA (%F) |
| 25 mg tablet (po) | 3261.51 | 420.44 | 2.00 | 8.02 | 49.38 | 4.42 | 44.31 |
| 50 mg tablet (po) | 5206.55 | 665.89 | 2.00 | 10.18 | 79.28 | 5.29 | 35.37 |
| 100 mg tablet (po) | 7478.26 | 898.84 | 1.75 | 12.77 | 119.16 | 6.89 | 25.40 |
| 25 mg solution (po) | 3372.32 | 393.31 | 1.50 | 6.90 | 58.45 | 6.32 | 45.82 |
| 2 mg/kg solution (iv) | 6182.38 | 1158.52 | 0.25 | 0.33 | 1.90 | 4.07 | - |

As shown in Table 14 above, in the case of tablets, the drug absorption (AUCₗₐₛₜ) tended to increase as the administration dose increased. The maximum concentration absorption time (Tₘₐₓ) of the drug showed a range of 1-2 hr at each dose, and the average half-life (t_{1/2}) showed 4.42 hr, 5.29 hr, and 6.89 hr, showing a pattern in which the excretion rate slowed down as the administration dose increased.

When comparing the 25 mg/head tablet administration group and the liquid administration group, the average absorption (AUCₗₐₛₜ) of the tablet administration group reached 96.7% of that of the liquid administration group, proving that the oral formulation of the poorly soluble drug compound of chemical formula 1 was optimized in terms of solubilization and *in vivo* absorption.

In conclusion, the present inventors obtained a stable novel crystalline solid of the compound of chemical formula 1, and secured an oral formulation with excellent stability and oral absorption rate using it.

## Claims

1. A crystalline form of the compound of chemical formula 1, having a powder X-ray diffraction (PXRD) pattern comprising peaks at 2θ diffraction angles of: 5.3±0.2°, 7.5±0.2°, 10.1±0.2°, 10.6±0.2°, 11.5±0.2°, 14.3±0.2°, 14.7±0.2°, 15.0±0.2°, 15.5±0.2°, 16.0±0.2°, 16.3±0.2°, 16.5±0.2°, 19.1±0.2°, 19.3±0.2°, 19.7±0.2°, 20.3±0.2°, 20.6±0.2°, 21.4±0.2°, 21.8±0.2°, 22.6±0.2°, and 27.5±0.2°.

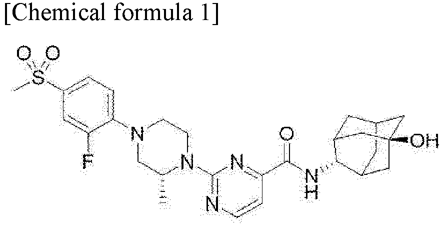

2. A method for preparing the crystalline form of the compound of chemical formula 1 according to claim 1, comprising
(S1) a step of dissolving the compound of chemical formula 1 in ethyl acetate; 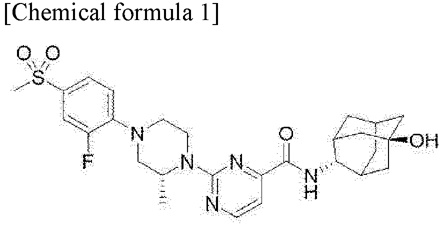
(S2) a step of adding t-butyl methyl ether to the ethyl acetate solution of step S1 to crystallize; and
(S3) a step of filtering the suspension of step S2 to obtain a crystalline compound of chemical formula 1.

3. A pharmaceutical preparation comprising particles prepared by drying water in which a compound of the chemical formula 1 is suspended and a hydrophilic polymer is dissolved.

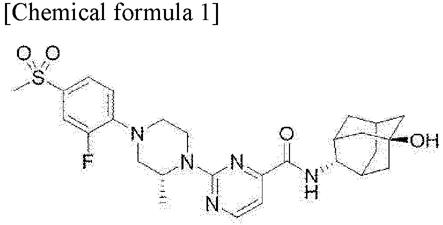

4. The pharmaceutical preparation according to claim 3, wherein the hydrophilic polymer is at least one selected from the group consisting of hypromellose, polyvinylpyrrolidone, polyvinyl alcohol, and hydroxypropyl cellulose.

5. The pharmaceutical preparation according to claim 4, wherein the hydrophilic polymer is hypromellose.

6. The pharmaceutical preparation according to claim 3, wherein the hydrophilic polymer is 30 to 80 wt% relative to the content of the compound of chemical formula 1.

7. The pharmaceutical preparation according to claim 6, wherein the hydrophilic polymer is 40 to 70 wt% relative to the content of the compound of chemical formula 1.

8. The pharmaceutical preparation according to claim 3, wherein the compound of chemical formula 1 is micronized.

9. The pharmaceutical preparation according to claim 8, wherein the micronized compound of chemical formula 1 has a d(50) of 5 µm or less.

10. The pharmaceutical preparation according to claim 9, wherein the micronized compound of chemical formula 1 has a d(50) of 2 µm or less.

11. The pharmaceutical preparation according to claim 3, wherein a surfactant is dissolved in the water.

12. The pharmaceutical preparation according to claim 11, wherein the surfactant is at least one selected from the group consisting of lauryl sulfate salt, poloxamer, glycerol monostearate, polyoxyethylene, and docusate salt.

13. The pharmaceutical preparation according to claim 12, wherein the surfactant is sodium lauryl sulfate.

14. The pharmaceutical preparation according to claim 11, wherein the surfactant is contained in an amount of 0.2 to 2 wt% based on the total weight of the pharmaceutical preparation.

15. The pharmaceutical preparation according to any one of claims 3 to 14, wherein the compound of chemical formula 1 has the crystalline form according to claim 1.

16. The pharmaceutical preparation according to any one of claims 3 to 14, wherein the pharmaceutical preparation is for oral use.
